Europäisches Patentamt

European Patent Office

Office européen des brevets

(19)

(11) Veröffentlichungsnummer: 0 056 266
A1

# (12) EUROPÄISCHE PATENTANMELDUNG

(21) Anmeldenummer: 82100106.2

(51) Int. Cl.³: A 61 B 17/00, B 23 B 31/04

(22) Anmeldetag: 09.01.82

(30) Priorität: 10.01.81 DE 3100512

(43) Veröffentlichungstag der Anmeldung: 21.07.82
Patentblatt 82/29

(84) Benannte Vertragsstaaten: CH FR GB IT LI

(71) Anmelder: Gustav Neuhäuser oHG
Präzisionswerkzeugfabrik, Hölderlinstrasse 2,
D-7130 Mühlacker-Enzberg (DE)

(72) Erfinder: Rauth, Roman, Werkstrasse 5,
D-7130 Mühlacker-Enzberg (DE)

(74) Vertreter: Hubbuch, Helmut Dipl.-Ing et al,
Patentanwälte Dr. Rudolf Bauer Dipl.-Ing. Helmut
Hubbuch Dipl.-Phys. Ulrich Twelmeier Westliche
Karl-Friedrich-Strasse 29-31, D-7530 Pforzheim (DE)

(54) Schnellwechselfutter.

(57) Die Erfindung betrifft ein Schnellwechselfutter zwischen Antriebsteilen (1, 2) und/oder Werkzeug mit Mitnehmerbacken (4) zur Drehmitnahme und Keileingriff (5) zur lösbaren Längs-verbindung, insbesondere für chirurgische Instrumente, welche gegenüber den bekannten Schnellwechselfutter in Form von Kupplungen sich einerseits ohne Werkzeug leicht lösen lässt und andererseits auch eine leichte Zerlegung in ihre Einzelteile zur Reinigung und Desinfektion sowie einen anschliessend einfachen Zusammenbau ermöglicht.

EP 0 056 266 A1

- 1 -

## Schnellwechselfutter

Die Erfindung bezieht sich auf Schnellwechselfutter zwischen Antriebsteilen und/oder Werkzeug mit Mitnehmerbacken zur Drehmitnahme und Keileingriff zur lösbaren Längsverbindung, insbesondere für chirurgische Instrumente.

Es sind solche Schnellwechselfutter in Form von Kupplungen mit Mitnehmerbacken zur Drehmitnahme und Keileingriff zur lösbaren Längsverbindung für Chirurgie bekannt, welche einerseits beim Öffnen leicht verklemmen können und andererseits sich auch nicht ohne Werkzeuge in die Einzelteile zerlegen lassen, wie dies vorallem für chirurgische Instrumente zur Reinigung und Desinfektion aber auch für chemische und pharmazeutische Rührwerke und dgl. mehr erwünscht ist.

Aufgabe der Erfindung ist nun vorallem für diese Zwecke ein Schnellwechselfutter zu schaffen, welches sich einerseits ohne Werkzeug leicht lösen lässt und andererseits auch eine leichte Zerlegung in ihre Einzelteile und einen anschliessend einfachen Zusammenbau ermöglicht.

Das Schnellwechselfutter nach dem Oberbegriff des Anspruchs 1 kennzeichnet sich gemäss der Erfindung hierzu dadurch, dass eine als Antriebsteil dienende Verriegelungshülse mit radial verschiebbarem Verriegelungsteil versehen ist, welcher von einem aufgeschobenen Verriegelungsring umfasst ist, der mittels Verriegelungskurve durch Verdrehen das Verriegelungsteil zur Verriegelung mit dem eingeführten Antriebsteil oder Werkzeug bringt, wobei in den Verriegelungsring einerseits eine Ringfeder eingreift, welche andererseits in einen Verschlussring greift, der mittels Verschlußstift an einer Öffnung an der Verriegelungshülse gegebenenfalls mit Ringeinsatz bis zu einer Ringnut einschieb- und unter Federwirkung gegenüber der Öffnung verriegelbar ist. Hierbei ist der Verriegelungsteil zweckmäßigerweise als Verriegelungskeil ausgebildet und die Verriegelungskurve steigt im Verriegelungsring zum Spannen des Verriegelungskeils flach gegen die Drehrichtung an. Hiermit werden die gestellten Forderungen gelöst und ein sicherer und ohne Werkzeug lösbarer Verschluß erreicht.

Weitere Einzelheiten des Schnellwechselfutters gemäss der Erfindung sind an Hand eines bevorzugten Ausführungsführungsbeispiels in der Zeichnung dargestellt und nachfolgend beschrieben und zwar zeigen:

Figur 1 und 5 den getrennten Querschnitt durch den Handgriff eines chirurgischen Instruments mit einerseitigem Schnell-

- 3 -

wechselfutter zwischen den Antriebsteilen und anderseitigem Schnellwechselfutter zwischen Antriebsteil
und Werkzeug und

Figur 2 bis 4 Einzeldarstellungen zu Figur 1
und

Figur 6 bis 8 Einzeldarstellungen zu Figur 5.

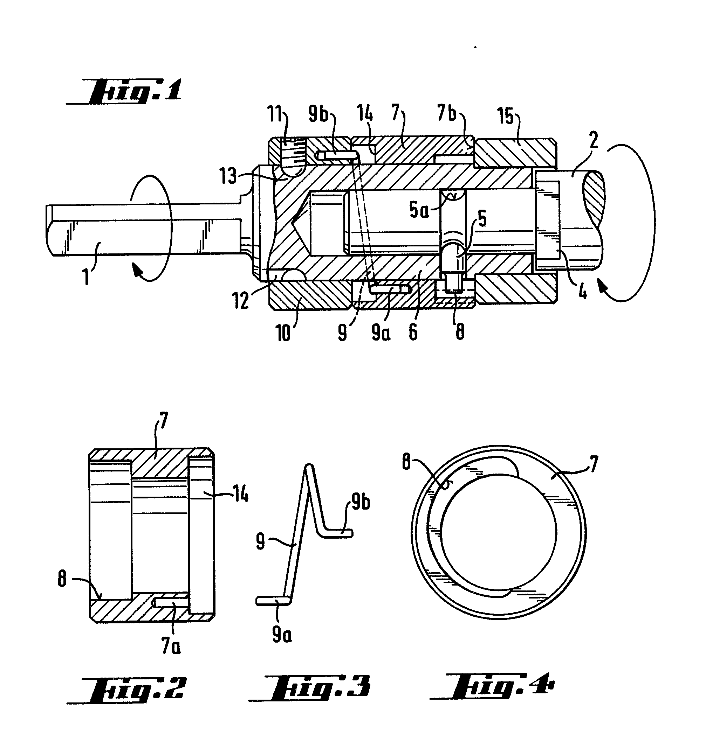

In Figur 1 und 2 bis 4 ist das Schnellwechselfutter
zwischen den Antriebsteilen 1 und 2 eines chirurgischen
Instruments dargestellt und in Figur 5 und 6 bis 8
das Schnellwechselfutter zwischen den Antriebsteilen 2
und Werkzeug 3 jeweils mit Mitnehmerbacken 4 zur
Drehmitnahme und Keileingriff 5 zur lösbaren Längsverbindung. Hierbei ist eine Verriegelungshülse 6
bzw. 6a als Antriebsteil 1 oder 2 mit radial verschiebbarem Verriegelungskeil 5 vorgesehen, welche
(6, 6a) von einem aufschiebbaren Verriegelungsring 7
umfasst ist, der (7) mittels Verriegelungskurve 8
durch Verdrehen den Keil 5 zur Verriegelung mit
dem eingeführten Antriebsteil 2 bzw. Werkzeug 3
bringt.

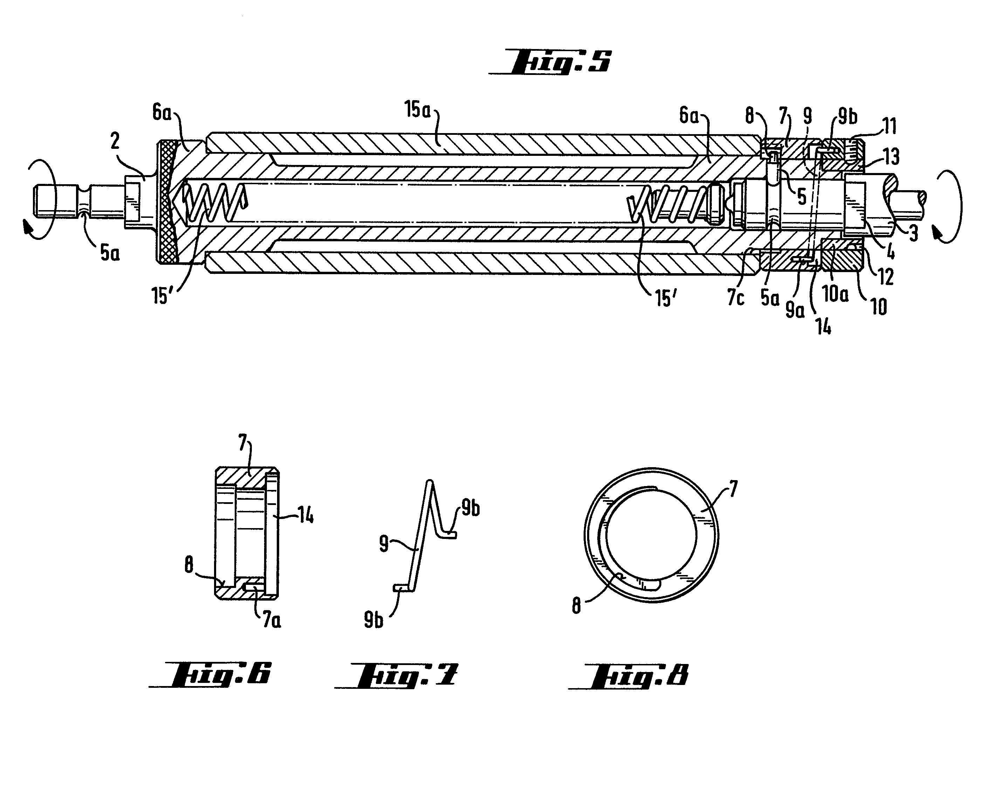

Zum Spannen des Verriegelungskeils 5 ist die Verriegelungskurve 8 im Verriegelungsring 7 flach gegen die
Drehrichtung ansteigend ausgebildet, wie das in
Figur 4 und 8 ersichtlich wird. In den Verriegelungsring 7 greift einerseits eine Ringfeder 9 ein,
wie in Figur 2 und 3 sowie Figur 6 und 7 ersichtlich
wird, welche (9) andererseits in einen Verschlussring 10 greift der mittels Verschlußstift 11 an einer Öffnung 12 an der Verriegelungshülse 6 (Figur 1) gegebenenfalls mit Ringeinsatz 10a (Figur 5) bis zu der Ringnut 13 einschieb- und unter Federwirkung gegenüber der Öffnung verriegelbar ist. Hierbei liegt die Ringfeder 9 entspr. Figur 3 und 7 in einer Stirnausnehmung 14 am Verriegelungsring 7 als Teilwindung und greift mit dem abgewinkelten Ende 9a einerseits in den Verriegelungsring 7 bei 7a und andererseits mit abgewinkeltem Ende 9b in den Verschlussring 10.

Nach Figur 1 ist der Verriegelungshülse 6 ein Abschlußstück 15 für die Mitnehmerbacken 4 fest aufgesetzt, welches (15) gleichzeitig als Anschlag 7b für den aufschiebbaren Verriegelungsring 7 dient.

Nach Figur 5 sitzt der Verriegelungshülse 6a ein Griffstück 15a drehbeweglich auf, welchem der aufschiebbare Verriegelungsring 7 folgt mit Anschlag 7c an der Hülse 6a. Bei durchgreifendem Werkzeuganschluß 3 weist hier der Verschlussring 10 am Ringeinsatz 10a die Mitnehmerbacken 4 auf. Bei Ausbildung der Verriegelungshülse 6a hier als Aufnahme für das durchgreifende Werkzeug nimmt diese (6a) einen Federbolzen 15 zur spielfreien Werkzeuglagerung auf.

Hiermit werden aus den Figuren 1 bis 8 und der Schilderung hierzu die beidseits vom Griffstück 15a beispielsweise für ein chirurgisches Instrument 3, welches hier nicht weiter dargestellt ist, das Schnellwechselfutter

0056266

- 5 -

zwischen dem Antriebsteil 1,2 bzw. dem Antriebsteil 2 und Werkzeug 3 gemäss der Erfindung ersichtlich. Wesentlich hierbei ist, dass dieselben schnell und sicher zu lösen und überdies leicht ohne Werkzeug in ihre Einzelteile zu zerlegen sind. Zur Lösung des Schnellwechselfutters ist jeweils der Verriegelungsring 7 in Drehrichtung zu verdrehen, wodurch die Verriegelungskurve 8 den Verriegelungskeil 5 bei 5a für den Antriebsteil 2 oder den Werkzeugabschluss 3 freigibt. Zum Zerlegen in die Einzelteile ist lediglich jeweils der Verschlussring 10 gegen Wirkung der Ringfeder 9 soweit zu verdrehen bis der in die Nut 13 der Verriegelungshülse 6 bzw. deren Ringeinsatz 10a greifende Verschlußstift 11 in Höhe der Öffnung 12 kommt und damit abzunehmen ist. Der Zusammenbau erfolgt in entsprechend umgekehrter Reihenfolge.

- 6 -

Patentansprüche:

1. Schnellwechselfutter zwischen Antriebsteilen und/ oder Werkzeug mit Mitnehmerbacken zur Drehmitnahme und Keileingriff zur lösbaren Längsverbindung, insbesondere für chirurgische Instrumente, dadurch gekennzeichnet, dass eine als Antriebsteil (1,2) dienende Verriegelungshülse (6,6a) mit radial verschiebbarem Verriegelungsteil (5) versehen ist, welcher (5) von einem aufgeschobenen Verriegelungsring (7) umfasst ist, der mittels Verriegelungskurve (8) durch Verdrehen das Verriegelungsteil (5) zur Verriegelung mit dem eingeführten Antriebsteil (2) oder Werkzeug (3) bringt, wobei in den Verriegelungsring (7) einerseits eine Ringfeder (9) eingreift, welche andererseits in einen Verschlussring (10) greift, der mittels Verschlußstift (11) an einer Öffnung (12) an der Verriegelungshülse (6) gegebenenfalls mit Ringeinsatz (10a) bis zu einer Ringnut (13) einschieb- und unter Federwirkung gegenüber der Öffnung verriegelbar ist.

2. Schnellwechselfutter nach Anspruch 1, dadurch gekennzeichnet, dass der Verriegelungsteil als Verriegelungskeil (5) ausgebildet ist und die Verriegelungskurve (8) im Verriegelungsring (7) zum Spannen des Verriegelungskeils (5) flach gegen die Drehrichtung ansteigt.

3. Schnellwechselfutter nach Anspruch 1, dadurch gekennzeichnet, dass die Ringfeder (9) in einer Stirnausnehmung (14) am Verriegelungsring (7) als Teilwindung liegt und einerseits mit abgewinkeltem Ende (9a) in den Verriegelungsring (7) und andererseits mit abgewinkeltem Ende (9b) in den Verschlussring (10) greift.

4. Schnellwechselfutter nach einem der Ansprüche 1 bis 3, dadurch gekennzeichnet, daß der Verriegelungshülse (6) ein Abschlußstück (15) für die Mitnehmerbacken (14) aufgesetzt ist, welches gleichzeitig als Anschlag (7b) für den aufschiebbaren Verriegelungsring (7) dient.

5. Schnellwechselfutter nach den Ansprüche 1 bis 3, dadurch gekennzeichnet, dass der Verriegelungshülse (6a) ein Griffstück (15a) drehbeweglich aufsitzt, welchem der aufschiebbare Verriegelungsring (7) folgt mit Anschlag (7c) an der Hülse (6a).

6. Schnellwechselfutter nach Anspruch 5, dadurch gekennzeichnet, dass bei durchgreifendem Werkzeuganschluss (3) der Verschlussring (10) am Ringeinsatz (10a) die Mitnehmerbacken (4) aufweist.

7. Schnellwechselfutter nach Anspruch 5 und 6, dadurch gekennzeichnet, dass bei Ausbildung der Verriegelungshülse (6a) als Aufnahme für das

0056266

- 8 -

durchgreifende Werkzeug (3) diese (6a) einen
Federbolzen (15) zur spielfreien Werkzeuglagerung aufnimmt.

0056266

0056266

**Fig. 5**

**Fig. 8**

**Fig. 7**

**Fig. 6**

Europäisches
Patentamt

**EUROPÄISCHER RECHERCHENBERICHT**

0056266
Nummer der Anmeldung

EP 82 10 0106

## EINSCHLÄGIGE DOKUMENTE

| Kategorie | Kennzeichnung des Dokuments mit Angabe, soweit erforderlich, der maßgeblichen Teile | betrifft Anspruch | KLASSIFIKATION DER ANMELDUNG (Int. Cl.³) |
|---|---|---|---|
| X | FR - A - 2 243 758 (BAHMÜLLER)<br><br>* Seite 3, Zeile 20 - Seite 6, Zeile 30; Figuren 1-3 * | <br><br>1,2 | A 61 B 17/00<br>B 23 B 31/04 |
| A | DE - A - 2 243 643 (GANNER)<br><br>* Seite 6, Zeile 23 - Seite 7, Zeile 24; Figuren 4,5 * | <br><br>1,2,4 | |
| A | GB - A - 15 717 A.D. 1912 (APPLEBY)<br><br>* Seite 1, Zeile 20 - Seite 2, Zeile 17; Figuren 1,2 * | <br><br>1,2 | RECHERCHIERTE SACHGEBIETE (Int Cl.³)<br><br>A 61 B<br>A 61 C<br>B 23 B |
| A | DE - A - 651 755 (BERGER)<br><br>* Seite 1, Zeile 31 - Seite 2, Zeile 65; Figuren 1-5 * | <br><br>1,5,7 | |

----------

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| DEN HAAG | 26-03-1982 | BARTLETT |

EPA form 1503.1   06.78